# EUROPEAN PATENT APPLICATION

(11) **EP 3 320 828 A1**
(43) Date of publication of application: **16.05.2018**
(21) Application number: 17196947.0
(22) Date of filing: 17.06.2015
(51) Int. Cl.: A61B 1/00, A61B 1/018

(54) **APPARATUS AND METHOD FOR COUPLING BETWEEN A COLONOSCOPE AND ADD-ON TUBES**

(30) Priority: 17.06.2014 US 201462012997 P; 20.11.2014 WO PCT/IL2014/051014; 27.05.2015 US 201514722400
(62) Divisional of application: 15735746.8
(71) Applicant: Motus GI Medical Technologies Ltd., 3902638 Tirat HaCarmel (IL)
(72) Inventor: HASSIDOV, Noam, 2521300 Doar-Na Oshrat (IL); SHTUL, Boris, 2907900 Kiryat-Yam (IL); KOCHAVI, Eyal, 3452620 Haifa (IL); ARNON, Tzach, 2018000 Doar-Na Misgav (IL); LULEKO, Koby, 2019600 Eshchar (IL); BLECHER, Dan, 5250451 Ramat-Gan (IL)
(74) Representative: Kramer, Dani

(57) **Abstract**

A sleeve assembly is described for coupling a colonoscope insertion tube to an add-on tube. In some embodiments, the sleeve assembly comprises an inner sleeve defining an elongated lumen with an open end, and an having an expanded state sized to surroundingly receive at least 70% of a length of an endoscope insertion tube arranged longitudinally within the inner sleeve; an outer sleeve encircling the inner sleeve; and one or more add-on tubes extending longitudinally between the inner sleeve and the outer sleeve; wherein inner sleeve is collapsible from the expanded state to fit tightly over the received length of the endoscope insertion tube; and collapsing the inner sleeve from the expanded state brings the outer sleeve radially closer to the endoscope while allowing at least some movement of the add on tubes in respect to the endoscope.

## Description

### FIELD AND BACKGROUND OF THE INVENTION

The present invention, in some embodiments thereof, relates to a colon cleaning system, and more particularly, but not exclusively, to a device and method for coupling between a colonoscope and one or more add-on tubes.

U.S Publication No. 2012/0101336 A1 to Hirsch et al. discloses "An endoscopic system for use with an endoscope, including an irrigation tube that provides a flow of irrigation fluid for cleaning a body lumen, and a suction tube for sucking material from the body lumen, wherein the suction tube is connected to a branch connector and one branch of the branch connector is connected to a suction source tube which is connected to a suction source and another branch of the branch connector is connected to a vent tube, and wherein the suction source tube and the vent tube pass through a double pinch valve."

### SUMMARY OF THE INVENTION

According to an aspect of some embodiments, there is provided a sleeve assembly for coupling between a colonoscope insertion tube and one or more add-on tubes, the sleeve assembly comprising: an inner sleeve defining an elongated lumen with an open end, and an having an expanded state sized to surroundingly receive at least 70% of the length of the colonoscope insertion tube arranged longitudinally within the inner sleeve; an outer sleeve encircling the inner sleeve; and one or more add-on tubes extending longitudinally between the inner sleeve and the outer sleeve; wherein inner sleeve is collapsible from the expanded state to fit tightly over the received length of colonoscope insertion tube; and wherein the inner sleeve is coupled to the outer sleeve at one or more locations along the inner sleeve so that collapsing the inner sleeve from the expanded state brings the outer sleeve radially closer to the inner sleeve, approximating the one or more add-on tubes to the inner sleeve.

According to some embodiments, at least one of the outer sleeve and the inner sleeve is radially stretched in the expanded state, and sufficiently elastic to drive the collapse to the tight fit over the received length of colonoscope insertion tube.

According to some embodiments, the radial stretch in the expanded state comprises stretching which increases the diameter of the inner sleeve by at least 2 mm over an unstretched state of the elastic sleeve.

According to some embodiments, the unstretched state of the elastic sleeve has a diameter of less than 12 mm.

According to some embodiments, in the expanded state of the inner sleeve, a diameter of the inner sleeve is at least 10% larger than a diameter of the colonoscope insertion tube receivable within the inner sleeve.

According to some embodiments, in a relaxed collapsed state of the inner sleeve a diameter of the inner sleeve is at least 5% smaller than a diameter of a selected diameter of colonoscope insertion tube received within it, to tightly fit the insertion tube.

According to some embodiments, the inner sleeve is expandable in a radially outwards direction along at least 60% of the length of the inner sleeve.

According to some embodiments, the inner sleeve is expandable along its complete length.

According to some embodiments, the outer sleeve is expanded as a result of expansion of the inner sleeve.

According to some embodiments, an inner circumferential surface of the outer sleeve is attached to an outer circumferential surface of the inner sleeve in at least one region along the sleeves.

According to some embodiments, the stiffness at least the inner sleeve varies along the longitudinal extent of the elongated lumen of the inner sleeve.

According to some embodiments, at least one of the add-on tubes is suitable for irrigation of the colon, and at least one of the add-on tubes is suitable for evacuation of matter from the colon.

According to some embodiments, at least one of the add-on tubes comprises a sensor.

According to some embodiments, the sensor is a pressure sensor.

According to some embodiments, the outer sleeve is shaped as a continuous cylinder having a length at least as long as a colonoscope insertion tube received within the inner sleeve.

According to some embodiments, at least one of the inner sleeve and the outer sleeve is non-contiguous and is formed of a plurality of elements interconnected by an axially extending element.

According to some embodiments, the elements are one or more collinear sleeves.

According to some embodiments, a length of the inner sleeve is in a range between 80 and 165 cm.

According to some embodiments, the outer sleeve comprises at least one guide member shaped to align the add-on tubes with respect to a longitudinal axis of the inner sleeve, thereby aligning the add-on tubes with respect to a colonoscope insertion tube received within the sleeve.

According to some embodiments, an inner surface of the outer sleeve defines one or more elongated grooves for receiving the one or more add-on tubes, the grooves extending parallel to a longitudinal axis of the inner sleeve in which a colonoscope insertion tube is received or at an angle with respect to the longitudinal axis of the inner sleeve.

According to some embodiments, the sleeve assembly comprises polyurethane.

According to some embodiments, the sleeve assembly further comprises an external sleeve encircling the outer sleeve to prevent over expansion during set up of a colonoscope insertion tube and add-on tubes using the sleeve assembly.

According to some embodiments, at least a portion of the inner and outer sleeves is defines a viewing window to enable viewing scale marks on a colonoscope insertion tube.

According to some embodiments, at least a distal end of the sleeve assembly is flexible to increase compliance with tissue when the sleeve assembly is used within a colon.

According to some embodiments, migration of the add-on tubes with respect to the colonoscope insertion tube is prevented along at least 60% of the length of the sleeve assembly when the add-on tubes are approximated to the inner sleeve.

According to some embodiments, the outer sleeve comprises a first outer layer adapted for contacting the walls of a colon when the sleeve is inserted to the colon, and a second inner layer holding the add-on tubes to the inner sleeve; the outer layer comprising a material having a friction coefficient smaller than a friction coefficient of a material of the inner layer.

According to some embodiments, the coupling of the outer sleeve to the inner sleeve comprises mutual attachment extending longitudinally along at least 50% of the length of the sleeve assembly.

According to an aspect of some embodiments of the invention, there is provided a system for coupling between a colonoscope insertion tube and one or more add-on tubes, comprising: a sleeve assembly; and an inflation module connectable to the sleeve assembly, the inflation module is configured to inflate at least a portion of the sleeve assembly to expand it.

According to some embodiments, the inflation module comprises an air pump and a regulator.

According to some embodiments, a proximal end of the sleeve assembly comprises a housing structured to couple between the sleeve assembly and the inflation module, the housing comprising a valve.

According to some embodiments, the housing is connected to a knob, the knob movable for selecting an orientation of the add-on tubes with respect to a handle of a colonoscope when an insertion tube of the colonoscope is positioned within the inner sleeve.

According to some embodiments, the system further comprises a jig shaped to receive at least a portion of the sleeve assembly.

According to some embodiments, the jig comprises one or more sensors for detecting at least one of a lengthwise positioning of a colonoscope insertion tube with respect to the sleeve assembly, and a positioning of the add-on tubes with respect to the colonoscope insertion tube.

According to some embodiments, the jig is serves as a packaging of the sleeve assembly.

According to some embodiments, the jig is telescopic and is expandable to at least a length of a colonoscope insertion tube received within the inner sleeve.

According to an aspect of some embodiments, there is provided a method for coupling between a colonoscope insertion tube and one or more add-on tubes, comprising: providing a sleeve assembly having at least one lumenal wall defining a longitudinally extending lumen and including at least one add-on tube also passing along the longitudinal extent of the sleeve assembly; positioning a colonoscope insertion tube in the longitudinally extending lumen; collapsing the sleeve assembly so that the add-on tubes are approximated radially closer to the colonoscope insertion tube, the sleeve assembly maintaining the add-on tubes in the approximated position with respect to the colonoscope insertion tube.

According to some embodiments, the method further comprises expanding an inner diameter of at least a portion of the sleeve before positioning the colonoscope insertion tube.

According to some embodiments, the expanding comprises inflating the sleeve.

According to some embodiments, the inflating comprises inflating with at least one of air and liquid.

According to some embodiments, the sleeve assembly comprises an inner sleeve surrounded by an outer sleeve, and wherein the expanding comprises reducing a volume of a lumen defined between outer walls of the inner sleeve and inner walls of the outer sleeve.

According to some embodiments, the reducing a volume comprises removing material from the lumen.

According to some embodiments, the removing comprises suctioning air out of the lumen.

According to some embodiments, the inner sleeve is expanded to a diameter larger than a diameter of the colonoscope insertion tube to reduce friction between the colonoscope insertion tube and walls of the inner sleeve during the positioning of the colonoscope insertion tube in the inner sleeve.

According to some embodiments, the expanding comprises stretching the sleeve along the longitudinal extent, and the collapsing is such that longitudinal tension remains in the at least one lumenal wall of the sleeve assembly after the add-on-tubes are approximated.

According to some embodiments, the collapsing fixates a positioning of the add-on tubes relative to the colonoscope insertion tube.

According to some embodiments, the method further comprises orienting the add-on tubes with respect to the inner sleeve during the collapsing.

According to some embodiments, the method further comprises removing the colonoscope insertion tube from the sleeve assembly by pulling on a thread which extends along the sleeve assembly and protrudes from a distal or proximal end of the sleeve assembly, to tear open the sleeve.

According to some embodiments, the method further comprises threading the one or more add-on tubes to extend longitudinally within a lumen defined by the sleeve assembly.

According to some embodiments, the one or more add-on tubes are integrated in the sleeve assembly.

According to some embodiments, the collapsing comprises bringing the add-on tubes radially closer to the colonoscope insertion tube.

According to some embodiments, the positioning comprises reducing friction between the colonoscope insertion tube and the sleeve assembly by at least one of: lubricating an inner surface of the sleeve assembly which defines a lumen in which the colonoscope insertion tube is received, lubricating the colonoscope insertion tube, and coating the inner surface of the sleeve assembly with a polymer based material suitable for reducing friction.

According to an aspect of some embodiments, there is provided an apparatus for coupling between a colonoscope insertion tube and one or more add-on tubes, the apparatus comprising: a sleeve defining an elongated lumen with an open end, and an expanded state sized to surroundingly receive at least 70% of the length of the colonoscope insertion tube arranged longitudinally within the sleeve; and one or more add-on tubes coupled to the sleeve across or more extensions distributed circumferentially around the sleeve; wherein an add-on tube coupled to the sleeve by one of the extensions extends alongside the sleeve for at least 70% of a length of the sleeve.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

Implementation of the method and/or system of embodiments of the invention can involve performing or completing selected tasks manually, automatically, or a combination thereof. Moreover, according to actual instrumentation and equipment of embodiments of the method and/or system of the invention, several selected tasks could be implemented by hardware, by software or by firmware or by a combination thereof using an operating system.

For example, hardware for performing selected tasks according to embodiments of the invention could be implemented as a chip or a circuit. As software, selected tasks according to embodiments of the invention could be implemented as a plurality of software instructions being executed by a computer using any suitable operating system. In an exemplary embodiment of the invention, one or more tasks according to exemplary embodiments of method and/or system as described herein are performed by a data processor, such as a computing platform for executing a plurality of instructions. Optionally, the data processor includes a volatile memory for storing instructions and/or data and/or a non-volatile storage, for example, a magnetic hard-disk and/or removable media, for storing instructions and/or data.

Optionally, a network connection is provided as well. A display and/or a user input device such as a keyboard or mouse are optionally provided as well.

### BRIEF DESCRIPTION OF THE SEVERAL VIEWS OF THE DRAWINGS

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
FIG. 1 is a flowchart of a general method for coupling between a colonoscope and one or more add-on tubes, according to some embodiments of the invention;
FIG. 2 is a flowchart of an exemplary method for coupling between a colonoscope and one or more add-on tubes using a sleeve assembly, according to some embodiments of the invention;
FIGs. 3A-3B show longitudinal (*Figure 3A*) and transverse (*Figure 3B*) cross sections of an exemplary sleeve assembly, according to some embodiments of the invention;
FIG. 4 is a schematic diagram of a system comprising an inflation module for coupling between a colonoscope and one or more add-on tubes, according to some embodiments of the invention;
FIG. 5 is an exemplary configuration of a sleeve assembly comprising one or more add-on tubes and configured for receiving a colonoscope, according to some embodiments of the invention;
FIGs. 6A-C are drawings of a set up procedure for coupling between a colonoscope and one or more add-on tubes using a sleeve assembly, according to some embodiments of the invention;
FIG. 7 is a drawing of a colonoscope coupled to one or more add-on tubes by a sleeve, according to some embodiments of the invention;
FIGs. 8A-8B show a side view (*Figure* 8*A*) and a front view (*Figure 8B*) of a distal housing of a sleeve assembly, according to some embodiments of the invention;
FIGs. 9A-9F show various schematic configurations for positioning one or more add-on tubes with respect to a colonoscope, according to some embodiments of the invention;
FIGs. 10A-10B are various alternative configurations of a sleeve, according to some embodiments of the invention;
FIG. 11 shows a colonoscopy system in which the colonoscope's insertion tube is coupled to a plurality of add-on tubes by a sleeve, according to some embodiments of the invention;
FIGs. 12A-12B illustrate an exemplary jig for setting up the colonoscope and add-on tubes assembly, according to some embodiments of the invention; and
FIG. 13 illustrates an exemplary mechanism for detaching the add-on tubes from the colonoscope, according to some embodiments of the invention.

### DESCRIPTION OF SPECIFIC EMBODIMENTS OF THE INVENTION

The present invention, in some embodiments thereof, relates to a colon cleaning system, and more particularly, but not exclusively, to a device and method for coupling between a colonoscope and one or more add-on tubes, configured for insertion into the colon. Optionally, a coupling is formed between an insertion tube of a colonoscope, and at least a portion of the length of one or more add-on tubes. In some embodiments, the add-on tubes comprise tubes for irrigation of the colon, tubes for evacuation of matter from the colon, and/or tubes configured for sensing within the colon, such as sensing of pressure. In some embodiments, the one or more add-on tubes and colonoscope are configured to be maneuvered together within a colon.

An aspect of some embodiments relates to coupling between a colonoscope and one or more add-on tubes. Optionally, the colonoscope is coupled to the add-on tubes outside the body, and the add-on tubes are held to the colonoscope for example by a sleeve as described herein, so that the colonoscope and tubes can be moved together.

In some embodiments, a system for coupling between the colonoscope and add-on tubes comprises a collapsible sleeve, which, when collapsed to a relaxed state, is configured to fit tightly over the colonoscope and to hold the add-on tubes to the colonoscope. In some embodiments, collapsing of the sleeve brings the one or more add-on tubes radially closer to the colonoscope. Optionally, the collapsed sleeve attaches between the colonoscope and tubes along at least a portion of the colonoscope, for example along a total of 60%, 70%, 80% or intermediate, larger or smaller percentages of the colonoscope's length. Optionally, the tubes and the colonoscope are attached at a plurality of spaced apart segments.

In some embodiments, collapsing of the sleeve comprises removing air from the sleeve. Optionally, not all parts of the sleeve are collapsed at the same time, for example a distal end of the sleeve is collapsed before a proximal end. Alternatively, the sleeve is collapsed at once. Optionally, the sleeve is collapsed by removal of a cap, for example from a distal end of the sleeve, thus enabling air to flow out of the sleeve.

In some embodiments, air bubbles which may form, for example, between the colonoscope and the sleeve are removed, for example by applying vacuum following the collapsing.

In some embodiments, in a non-collapsed state of the sleeve, a diameter of the sleeve is at least 5%, 10%, 20% or intermediate, larger or smaller percentages larger than a diameter of a colonoscope intended to be positioned within the sleeve.

Optionally, in a non-collapsed state of the sleeve, the sleeve is expanded in a radially outwards direction, for example by inflation. A potential advantage of an expanded sleeve may include facilitating insertion of the colonoscope into the sleeve during set up, for example by threading the colonoscope into the sleeve.

In some embodiments, the sleeve is a cylinder comprising one or more internal lumens. Optionally, the one or more lumens extend between a proximal end and a distal end of the sleeve. In some embodiments, the sleeve extends to a length at least as long as a colonoscope received within it. Optionally, the sleeve is continuous.

Alternatively, the sleeve is non-continuous, and is formed of, for example, a plurality of tubular segments or rings.

Optionally, the sleeve is configured for aligning between the colonoscope and the add-on tubes, for example aligning the add-on tubes with respect to a longitudinal axis of the colonoscope. In some embodiments, the sleeve is shaped to hold the tubes wound around the colonoscope. Additionally or alternatively, the sleeve holds the tubes aligned in parallel to the colonoscope. In some embodiments, the shaping of the sleeve for tube alignment comprises providing the sleeve with one or more guide members; for example, a closed channel, a guide-wall, and/or another form of longitudinally extending (optionally, helical or partially helical) protrusion and/or groove which provides a track along which a tube is constrained to extend.

In some embodiments, a coupling between the colonoscope and add-on tubes is formed with some degree of freedom, for example allowing, to a certain extent, axial sliding of the add-on tubes with respect to the colonoscope or vice versa, allowing, to a certain extent, angular rotation of the tubes with respect to the colonoscope and/or allowing, to a certain extent and for no more than, for example, 30% of the colonoscope's length, a radial gap between the colonoscope and add-on tubes. A potential advantage of a coupling with a certain degree of freedom may include reducing a rigidity of the complete colonoscope and add-on tubes assembly, which may facilitate maneuvering in the colon. In some embodiments, a rigidity is reduced by using the sleeve to couple between the colonoscope and add-on tubes along only a portion of the colonoscope's length, for example along 40%, 60%, 85% or intermediate, larger or smaller percentages of the length, and using other coupling means to couple the rest of the colonoscope's length to the tubes, for example using a plurality of tape wraps.

In some embodiments, a sleeve assembly is provided, for example including an inner sleeve positioned within an outer sleeve. Optionally, the inner sleeve is attached to the outer sleeve along at least a circumferential segment of the sleeves' walls. In some embodiments, the inner sleeve is configured to receive a colonoscope, and a lumen between the inner sleeve and the outer sleeve includes the add-on tubes to be coupled to the colonoscope. Optionally, the one or more add-on tubes are an integrated part of the sleeve. Additionally or alternatively, the one or more add-on tubes are passed within the lumen.

In some embodiments, expansion of the sleeve assembly or a portion of it such as the inner sleeve is obtained by inflation. Optionally, inflation is carried out by connecting the sleeve assembly, for example through a valve, to an inflation module.

Optionally, the inflation module comprises a pump and a pressure regulator. In some embodiments, the inner sleeve is inflated, and the outer sleeve, which encircles the inner sleeve, is caused to expand as well. In some embodiments, inflation causes overstretching of the inner and/or outer sleeve longitudinally, so that when collapsed, ripples are reduced. Optionally, inflation is performed to detect tears or other defects in the sleeve.

In some embodiments, the inner and/or outer sleeves are formed with varying degrees of stiffness, for example different stiffness properties for different longitudinal sections of the sleeve, to accommodate portions of the colonoscope having different flexibility properties.

In some embodiments, the add-on tubes are oriented with respect to a handle of the colonoscope, for example so that maneuvering of the colonoscope within the body is least affected by the add-on tubes.

In some embodiments, the system comprises a jig for use during set up of the sleeve and add-on tubes. Optionally, the jig is shaped to straighten the sleeve assembly during the attachment process. Optionally, the jig is shaped and/or sized to prevent overexpansion of the sleeve assembly. Optionally, the jig is shaped to define a track for threading of the colonoscope and/or the add-on tubes, so that they are positioned at a designated orientation with respect to each other.

In some embodiments, the jig is a telescopic track which can be expanded to at least a length of the colonoscope. In some embodiments, a packaging of the sleeve serves as the jig. In some embodiments, an external sleeve, for example a rigid sleeve, serves as the jig. Optionally, the rigid external sleeve reduces or prevents over expansion. In some embodiments, a colonoscope's works station is formed with a jig on which the colonoscope and add-on tubes can be set up.

In some embodiments, the jig comprises one or more sensors, for example for detecting a pressure level in the sleeve assembly, detecting a positioning of the colonoscope and/or add-on tubes, and/or detecting a fit of the collapsed sleeve assembly to the colonoscope.

Additionally or alternatively, means other than the jig are used for aligning the colonoscope and the add-on tubes, for example, a bridge shape element which is slid over the sleeve assembly to straighten it, or vertically hanging the sleeve assembly, utilizing gravity to straighten it.

In some embodiments, a disposable sleeve with pre-attached add-on tubes is provided, and a colonoscope is threaded through to be coupled to the tubes.

It is noted that in some embodiments, the apparatuses and/or methods described herein may be applied for coupling between an endoscope and one or more add-on tubes.

As used herein, the term "proximal" may include any portion of the colonoscope, add-on tubes and/or other components of the colonoscopy system which are closer to a user manipulated end of the device, and may be configured to remain outside the body; the term "distal" may include any portion of the colonoscope, add-on tubes and/or other components of the colonoscopy system which are closer to an end configured for insertion into the colon. Orientation of other components described herein may be defined with respect to the proximal and distal ends of the system.

As referred to herein, a typical colonoscope includes one or more of an insertion tube or a portion of the insertion tube, a Y-connector, an umbilical cord, a handle. As a general note, when a colonoscope is referred to in the context of coupling to one or more add-on tubes, the term "colonoscope" may refer to at least a portion of an insertion tube of the colonoscope.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

Before explaining at least one embodiment of the invention in detail, it is to be understood that the invention is not necessarily limited in its application to the details set forth in the following description or exemplified by the Examples. The invention is capable of other embodiments or of being practiced or carried out in various ways.

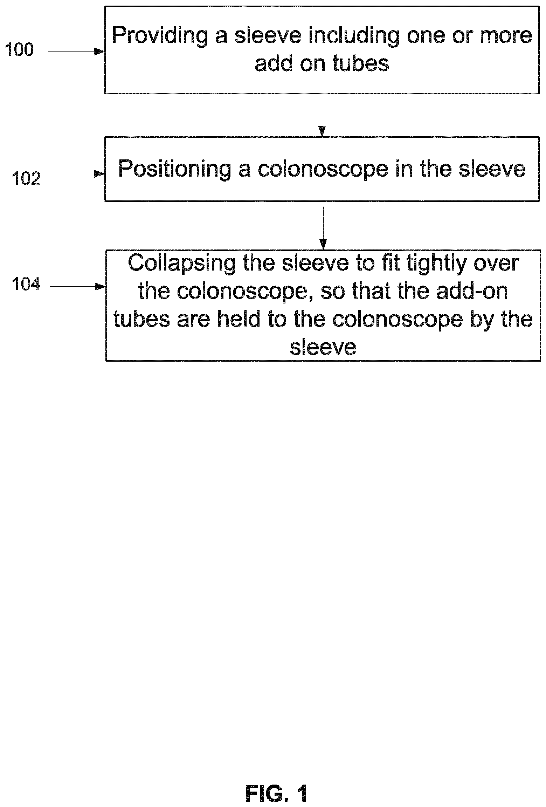

Referring now to the drawings, *Figure 1* is a flowchart of a general method for coupling between a colonoscope and one or more add-on tubes, according to some embodiments of the invention.

In some embodiments, the method is performed prior to a colonoscopy procedure, for example by a physician or other medical and/or technical personnel. In some embodiments, the method is performed externally to the body, to couple between a colonoscope and one or more add-on tubes, which are intended for insertion into a patient's colon.

In some embodiments, a sleeve is provided **(100).** Optionally, the sleeve comprises one or more add-on tubes. Optionally, the add-on tubes are an integrated part of the sleeve, for example attached to an inner wall of the sleeve. Additionally or alternatively, one or more tubes are inserted into the sleeve. Optionally, the tubes are passed along a length of the sleeve.

In some embodiments, the add-on tubes comprise one or more of: a tube for irrigation of the colon; a tube for evacuation of matter from the colon, such as fecal matter; a tube configured for sensing within the colon, for example a tube comprising a pressure sensor at its distal end; a tube configured to deliver a tool into the colon.

Optionally, the one or more add-on tubes are connected to a work station, for example at their proximal end. For example, in some embodiments, an evacuation tube is connected to a vacuum source. In some embodiments, an irrigation tube is connected to a liquid tank. In some embodiments, a pressure sensing tube is connected a vacuum source.

In some embodiments, the sleeve is disposable. Optionally, the add-on tubes are disposable.

In some embodiments, a colonoscope is positioned within the sleeve **(102).**

Optionally, the colonoscope is threaded into the sleeve. Optionally, the colonoscope is rotated during insertion into the sleeve. In some embodiments, the sleeve may comprise an aperture through which the colonoscope can be placed, for example an elongated slot which can be closed once the colonoscope has been placed within the sleeve. Optionally, friction at the interface between the colonoscope and the sleeve's walls is reduced, for example to reduce the amount of force which needs to be exerted on the colonoscope to pass it through the sleeve. Optionally, friction is reduced by a sleeve having a diameter which, at least along some segments of the sleeve and/or at certain times, for example prior to collapsing of the sleeve, is larger than a diameter of the colonoscope. Optionally, an oversized diameter of the sleeve is obtained by inflating the sleeve or portions of it, for example with air. Additionally or alternatively, friction is reduced by lubricating the colonoscope and/or the inner walls of the sleeve, for example using Vaseline. Additionally or alternatively, friction is reduced by a coating on the inner walls of the sleeve which is effective to reduce friction, for example comprising a polymer material.

In some embodiments, insertion of the colonoscope into the sleeve is aided, for example, by a jig. Optionally, for example as will be further described herein, the jig defines a track for positioning of the sleeve during insertion of the colonoscope. In some embodiments, the jig straightens the sleeve, to facilitate insertion of the colonoscope through.

In some embodiments, after insertion of the colonoscope into the sleeve, the sleeve is collapsed **(104).** Optionally, collapsing comprises removing air from a lumen of the sleeve, for example by suctioning and/or by letting air flow out of the sleeve.

Optionally, collapsing brings the one or more add-on tubes closer to the colonoscope, for example radially closer. In some embodiments, the sleeve holds the tubes aligned parallel to a longitudinal axis of the colonoscope. Additionally or alternatively, the sleeve holds the tubes at an angle relative to a longitudinal axis of the colonoscope. Optionally, the tubes are wound around the colonoscope.

In some embodiments, the collapsed sleeve restricts movement of the add-on tubes with respect to the colonoscope or vice versa, for example radial movement inward and/or outward with respect to each other, axial movement with respect to each other, and/or rotational movement with respect to each other. Additionally or alternatively, the collapsed sleeve is arranged to provide some freedom of movement.

Optionally, the sleeve is arranged to restrict movement along some portions of the sleeve, and allow movement, for example to a certain extent, at other portions of the sleeve. For example, in some embodiments, the sleeve is arranged to allow sliding of the tubes with respect to the colonoscope. Additionally or alternatively, the sleeve is arranged to allow one or more radial gaps between the colonoscope and tubes, which may increase or decrease in size. Optionally, the radial boundary of movement is selected to maintain the complete coupled assembly of the colonoscope and tubes under a certain diameter, for example a diameter ranging between 12-25 mm, such as 14 mm, 18 mm, 24 mm or intermediate, larger or smaller diameters. A potential advantage of enabling movement of the tubes and/or colonoscope with respect to each other, for example to a certain extent, may include reducing a rigidity of the coupled assembly. Reducing a rigidity may facilitate maneuvering of the complete assembly inside the colon.

In some embodiments, for example following use, a colonoscope is removed from the sleeve. Optionally, the colonoscope is removed by cutting the sleeve.

Optionally, removing the colonoscope comprises cutting a slot, for example at a distal end of the sleeve, and tearing the sleeve open. In some embodiments, a thin thread, for example formed of metal or nylon, is previously passed within the sleeve, for example the thread is inserted along with the colonoscope, and the sleeve can be torn open by pulling on an end of the thread which extends beyond a proximal and/or distal opening of the sleeve. Optionally, the thread is integrated within the sleeve.

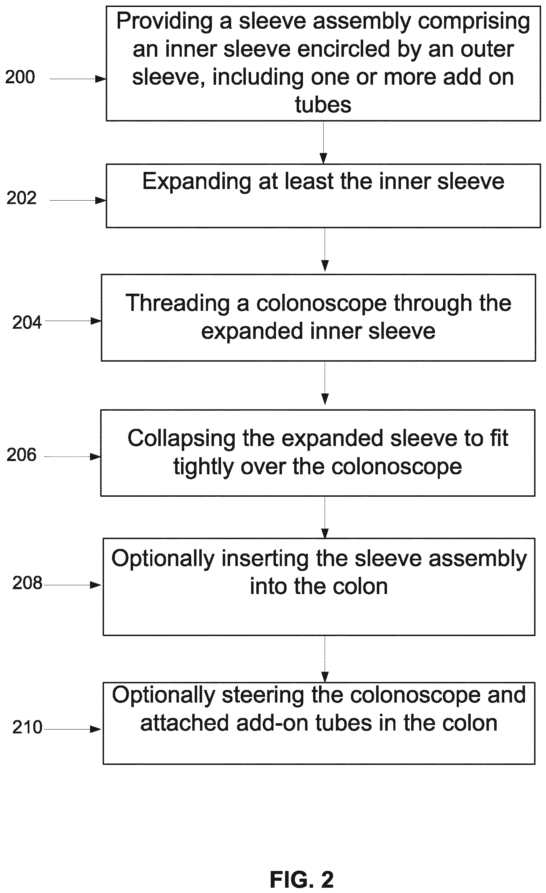

*Figure 2* is a flowchart of an exemplary method for coupling between a colonoscope and one or more add-on tubes using a sleeve assembly comprising an inner sleeve and an outer sleeve, according to some embodiments of the invention.

In some embodiments, a sleeve assembly comprising an inner sleeve encircled by an outer sleeve is provided **(200).** Optionally, one or more add-on tubes are an integrated part of the sleeve assembly, for example positioned at a lumen between the inner and outer sleeve. Additionally or alternatively, one or more add-on tubes are passed within the sleeve.

In some embodiments, one or more portions of the sleeve assembly are expanded. Optionally, the sleeve or one or more segments of it are expanded in a radial direction. In some embodiments, a length of the sleeve is extended in a proximal direction and/or in a distal direction.

In some embodiments, the inner sleeve is expanded **(202).** In some embodiments, expansion is obtained by inflating the inner sleeve, for example with air.

Additionally or alternatively, the inner sleeve is inflated with fluid. In some embodiments, the inner sleeve is expanded by removal of material, for example from a lumen between the inner and outer sleeve. Optionally, air is removed from the lumen between the sleeves, for example by suctioning, causing the inner sleeve to radially expand.

In some embodiments, the inner sleeve is expanded to a diameter similar to or larger than a diameter of a colonoscope intended to be received within the sleeve, for example 5%, 10%, 20%, 30%, or intermediate, larger or smaller percentages larger than a diameter of the colonoscope. Optionally, the walls of the inner sleeve exert force on the walls of the outer sleeve, causing it to expand as well.

In some embodiments, a material of the inner and/or outer sleeve is selected to be elastic enough to provide expansion. Alternatively, the outer sleeve is formed of a rigid material. Optionally, a rigid outer sleeve restricts the expansion of the inner sleeve.

In some embodiments, a colonoscope is inserted into the expanded inner sleeve **(204),** for example by threading. Optionally, the inner sleeve and/or outer sleeve are over stretched longitudinally, and may extend, for example, to a distance past a head of the colonoscope, for example extending 1 mm, 2 mm, 5 mm, 10 mm, 1 cm, 5 cm 10 cm or intermediate, larger or smaller distances past a distal head of the colonoscope. A potential advantage of over stretching may include reducing or preventing ripples and/or kinks of the sleeve, for example after collapsing.

In some embodiments, at least the inner sleeve is collapsed **(206).** In some embodiments, collapsing includes removing air from the sleeve, for example by suctioning the air and/or by letting air out. Optionally, the outer sleeve, due to its elastic properties, is collapsed as well. Optionally, the collapsed inner sleeve fits tightly over the colonoscope. A tight fit may be obtained, for example, by an inner sleeve having a collapsed diameter smaller than that of the colonoscope, for example 5%, 10%, 15%, 30% or intermediate, larger or smaller percentages smaller than a diameter of the colonoscope. In some embodiments, an expanded state of a sleeve lumen, suitable in diameter to receive a colonoscope insertion tube, comprises elastic stretching of at least 2 mm in lumenal diameter over an unstretched state. The elastic stretching is optionally of the inner sleeve, outer sleeve, or both.

In some embodiments, the colonoscope and attached add-on tubes which are held together by the sleeve assembly are inserted into the colon **(208).** Optionally, the colonoscope and tubes are steered together in the colon **(210).**

In some embodiments, if relative movement, to a certain extent, is allowed between one or more of the add-on tubes and the colonoscope, a user may adjust their relative positioning, for example by axially sliding an add-on tube, for example, an add-on tube configured for irrigation, to a distance beyond the colonoscope's distal head, such as to clear the colon area in front of the colonoscope's head. Optionally, the colonoscope's positioning in the sleeve can be adjusted in-vivo, for example by reinflating the sleeve, advancing or pulling the colonoscope to a desired positioning in the sleeve, and collapsing the sleeve. Optionally, when performed in-vivo, a pressure level of inflation of the sleeve is set to a level lower than a pressure level of ex-vivo inflation.

In some embodiments, for example after the colonoscope and add-on tubes are withdrawn from the colon, the colonoscope is removed from the sleeve. Optionally, the add-on tubes are removed from the sleeve. Optionally, the sleeve comprising the add-on tubes is disposed. Alternatively, the add-on tubes are removed, and the sleeve is disposed. Optionally, the add-on tubes are sterilized for additional use.

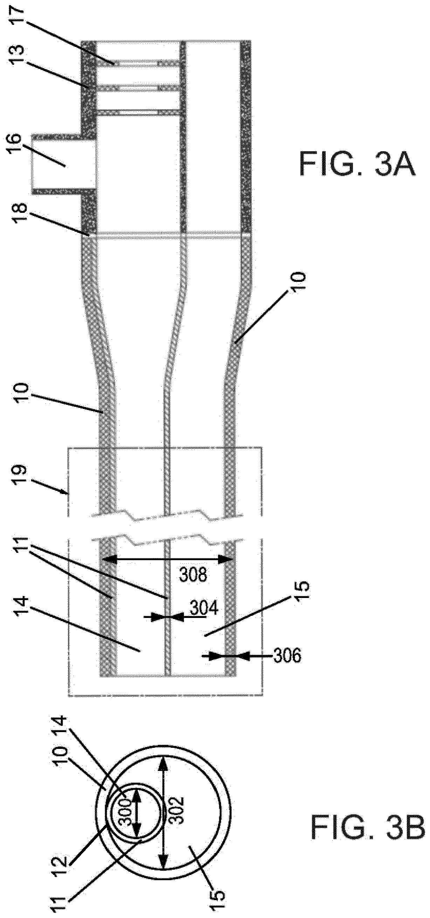

*Figures 3A-3B* show longitudinal (*Figure 3A*) and transverse (*Figure 3B*) cross sections of an exemplary sleeve assembly, according to some embodiments of the invention.

In some embodiments, the sleeve assembly comprises an inner sleeve **11,** adapted for receiving a colonoscope. Inner sleeve **11** is circumferentially surrounded by an outer sleeve **10,** including one or more add-on tubes. The exemplary configuration shown herein does not show add-on tubes, and those may be inserted into the sleeve, for example inserted into lumen **15** of outer sleeve **10,** such as between the walls of inner sleeve **11** and outer sleeve **10.** Alternatively, the add-on tubes are an integrated portion of the sleeve, for example, the tubes are formed during manufacturing of the sleeve.

Segment **19** of the sleeve assembly, for example as shown in *figure 3A**,* is intended for insertion into the colon. Optionally, the length of segment **19** is in a range between, for example, 20 cm - 200 cm, 50-165 cm, and/or 80-165 cm; for example, having a length of 50 cm, 150 cm, 165 cm, 180 cm or an intermediate, longer or shorter length.

In some embodiments, the inner sleeve and outer sleeve are attached to each other. For example, one or more circumferential segments of the wall of inner sleeve **11** are attached to the wall of outer sleeve **10.** Optionally, a coupling **12** between the sleeves (for example as shown in *figure 3B*) extends along 5%, 10%, 15%, 25%, 45% or intermediate, larger or smaller portions of the circumference of inner sleeve **11.** Optionally, the sleeves are attached to each other at more than one circumferential location, for example, 2, 3, 4, 5 or larger number of locations.

Additionally or alternatively, in some embodiments, coupling **12** comprises attachment along a line which is up to a few millimeters in width in the circumferential direction (for example, about 1 mm, 2 mm, 3 mm, 4 mm, 5 mm, or another greater, smaller, or intermediate width).

Optionally, coupling **12** extends continuously along a longitudinal axis of inner sleeve **11** and outer sleeve **10** for about 50%, 60%, 70%, 80%, 90%, or another greater, larger, or intermediate fraction of the overall length of segment **19** of the sleeve assembly, and/or of the sleeve assembly overall. Optionally, the extent of coupling **12** along the longitudinal axis is broken into a plurality of sub-segments.

In some embodiments, the longitudinal extent of the coupling excludes a section of the sleeve designated for covering a steering section of a colonoscope. This is a potential advantage to reduce interference with steering due, for example to asymmetric elastic properties induced by attachment between the sleeves.

In some embodiments, the coupling attachment 12 between the sleeves comprises, for example, welded attachment. Optionally, the sleeves are glued to each other. Additionally or alternatively, the sleeves are coupled to each other by other fastening means, such as flexible rings or wires. In some embodiments, coupling **12** includes a ratchet, which provides for, for example, relative movement of inner sleeve **11** within outer sleeve **10,** and/or, for example, for restricting an expansion of inner sleeve **11.**

In some embodiments, a diameter **300** of a lumen **14** of inner sleeve **11,** for example as shown in *figure 3B**,* in a non-expanded state, ranges between, for example, 8-12 mm, 3-10 mm, 5-18 mm, or intermediate, larger or smaller ranges. Optionally, diameter **300** is selected according to a diameter of a colonoscope, for example, diameter **300** can be 5%, 10%, 20% or intermediate, larger or smaller percentages smaller than a diameter of a colonoscope. In one example, for a colonoscope having a diameter of 12 mm, diameter **300** is 0.1-5 mm, 1-3 mm, 0.1-0.9 mm or intermediate, larger or smaller ranges smaller than the colonoscope's diameter in a non-expanded state.

In some embodiments, diameter **302** of lumen **15** of outer sleeve **10,** for example as shown in *figure 3B**,* ranges between, for example, 10-20 mm, such as 12 mm, 15 mm, 18 mm, or intermediate, larger or smaller diameters. Optionally, diameter **302** is selected according to a size and/or number of add-on tubes which lumen **15** accommodates.

In some embodiments, a thickness **304** of the wall of inner sleeve **11,** for example as shown in *figure 3A**,* is selected to be thick enough so as to reduce tearing by the colonoscope, for example during insertion of the colonoscope through, yet thin enough to reduce rigidity of the colonoscope. Optionally, thickness **304** ranges between 0.2-2 mm, such as 0.5 mm, 1 mm, 1.5 mm or intermediate, larger or smaller thicknesses.

In some embodiments, a thickness **306** of the wall of outer sleeve **10,** for example as shown in *figure 3A**,* is selected to be thin enough so as to reduce rigidity and/or to reduce a total diameter **308** of the sleeve assembly, for example as shown in *figure 3A**.* Optionally, a total diameter **308** of the sleeve assembly ranges between, for example, 13-25 mm, such as 15 mm, 20 mm, 24 mm or intermediate, larger or smaller diameters. Optionally, a thickness **306** of the wall of outer sleeve **10** ranges between 0.2-2 mm.

In some embodiments, the proximal ends of inner sleeve **11** and outer sleeve **10** are enclosed within a proximal housing **13,** for example as shown in *figure 3A**.*

Optionally, housing **13** is rigid. Optionally, housing **13** is made of, for example, polyurethane. Optionally, housing **13** connects between the sleeve assembly and the colonoscope's handle. Optionally, housing **13** connects between one or more components of the sleeve assembly, such as add-on tubes, and a workstation.

Optionally, housing **13** is rigid enough to transfer torsion towards a distal end of the sleeve assembly, for example during steering in the colon.

In some embodiments, housing **13** comprises one or more openings, such as opening **16.** Optionally, air and/or liquid used for inflating inner sleeve **11** are delivered through opening **16,** for example by connecting opening **16** to a pump.

Optionally, a valve is positioned at opening **16,** for controlling the flow in and/or out of the sleeve.

In some embodiments, one or more sealing rings **17,** for example as shown in *figure 3A**,* are positioned in the sleeve assembly, for example, positioned within inner sleeve **11.** Optionally, sealing rings **17** reduce or prevent leakage of air and/or liquid from the inflated sleeve, for example during insertion of the colonoscope through.

In some embodiments, the sleeve assembly is made of a flexible material, such as polyurethane (PU), Silicon. Optionally, the material is selected to be elastic enough to reduce rigidity of the complete colonoscope- add-on tubes assembly, enabling steering in the colon, and, on the other hand, the material is selected to be rigid enough to maintain the add-on tubes coupled to the colonoscope, and restrict movement of the tubes with respect to the colonoscope.

In some embodiments, at least a portion of the sleeve assembly, for example at least 40%, 60%, 80% or intermediate, larger or smaller percentages of the length of the sleeve, for example segment **19,** is made of a transparent material. Optionally, scale marks on a colonoscope are visible through the transparent sleeve. Optionally, a portion of the sleeve defines a viewing window through which the scale marks are visible.

In some embodiments, the sleeve assembly is manufactured using laser welding and/or extrusion techniques.

In some embodiments, outer sleeve **10** is coated by friction-reducing material such as Parylene C, which may facilitate insertion into the colon.

In some embodiments, outer sleeve **10** is formed of multiple layers of materials having different friction coefficients. In an example, an outer layer of sleeve **10** which may come in contact with the walls of the colon comprises a material having a smaller friction coefficient than the friction coefficient of a material forming an inner layer of sleeve **10.** Optionally, a multi layered sleeve such as double layered sleeve is manufactured using co-extrusion technology. A sleeve comprising two or more materials having different friction properties may be effective to reduce friction between the sleeve and the walls of the colon by an outer layer comprising a first friction coefficient which is smaller than a friction coefficient of an inner layer of the sleeve, which in turn may be effective to increase the contact strength when holding the add-on tubes to the colonoscope and/or to contribute to unified movement of the sleeve and the colonoscope positioned within it.

A sleeve, for example as described herein, is one example of an apparatus for coupling a colonoscope to one or more add-on tubes. It is noted that in some embodiments, an element having a general tubular geometry which is suitable for receiving a colonoscope, for example a spring element, may be used for coupling between the colonoscope and add-on tubes.

It is further noted that in some embodiments, a tubular element may comprise shape memory materials, for example nitinol. Optionally, collapsing and/or expanding of a tubular element may include applying stress, a change in temperature, an electric or magnetic field to induce changes in the shapeable material, causing the material to expand and/or collapse.

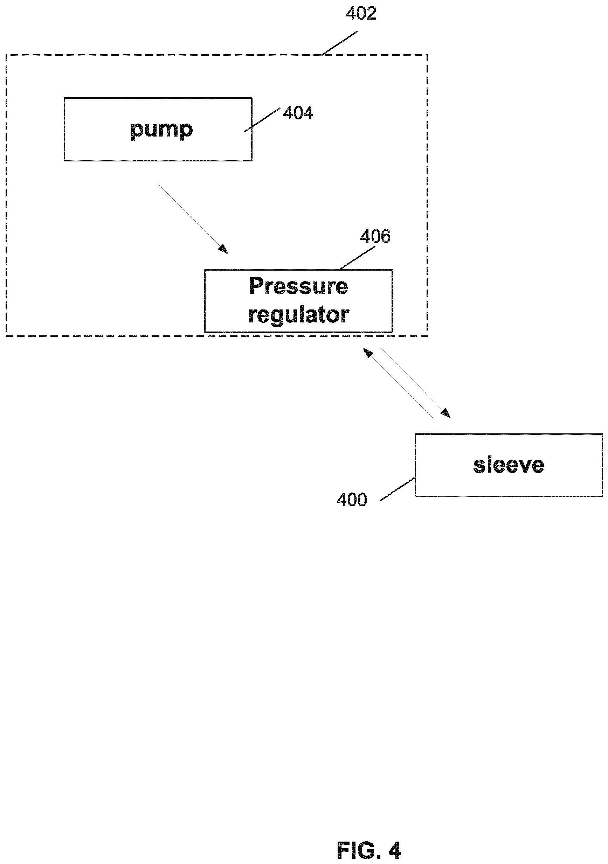

*Figure 4* is a schematic diagram of a system comprising an inflation module for coupling between a colonoscope and one or more add-on tubes, according to some embodiments of the invention.

In some embodiments, a sleeve **400** for example as described herein is connected to an inflation module **402.** Optionally, inflation module **402** comprises a pump **404,** such as an air pump. Optionally, inflation module **402** comprises a pressure regulator **406.**

In some embodiments, pump **404** is configured for supplying an air pressure of, for example 300-600 mbar, for example 300 mbar, 350 mbar, 450 mbar, 550 mbar or intermediate, higher or lower pressure levels. Optionally, pressure regulator **406** modifies the flow of air or liquid from pump **404.** Optionally, the pressure in the sleeve is dynamically adjusted by pressure regulator **406.**

In some embodiments, sleeve **400** (or a portion of it, such as an inner sleeve) is inflated to a maximal pressure of, for example, 300 mbar, 400 mbar, 350 mbar, or intermediate, higher or lower pressure levels. Optionally, different portions of the sleeve are inflated to different pressure levels. In some embodiments, selective inflation is performed. For example, gradual inflation of the sleeve (e.g. step wise inflation of the sleeve, one segment after another) is performed during insertion of the colonoscope through the sleeve. A potential advantage of selectively inflating portions may include reducing perforation of the sleeve, for example by the colonoscope. Another potential advantage may include obtaining a better fit of the sleeve to the colonoscope, after collapsing.

Optionally, sleeve **400** is inflated until reaching a diameter suitable for facilitating insertion of the colonoscope through.

In some embodiments, inflation module **402** is configured for applying vacuum to the sleeve, for example to remove air after positioning of the colonoscope in the sleeve to collapse the sleeve.

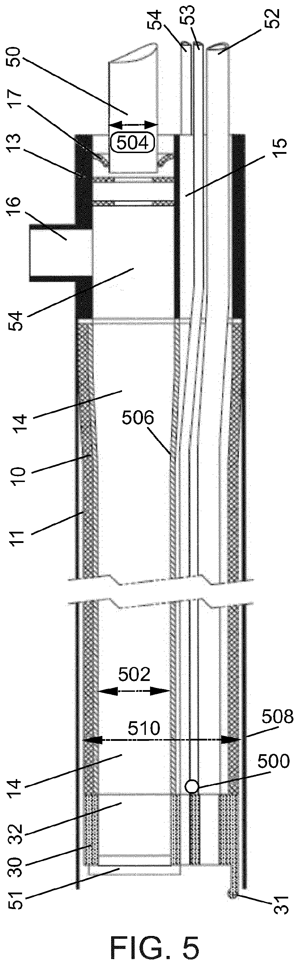

*Figure 5* shows an exemplary configuration of a sleeve assembly comprising one or more add-on tubes and configured for receiving a colonoscope to couple between the tubes and the colonoscope, according to some embodiments of the invention.

In some embodiments, a plurality of add-on tubes such as 2, 3, 4, 6, 9, or intermediate, larger or smaller number are comprised within the sleeve assembly. For example, as shown in this *figure, 3* add-on tubes **52, 53** and **54** are positioned within lumen **15** of outer sleeve **10.** Optionally, tubes **52, 53** and **54** vary in diameter and/or length, for example according to their function. In an example, tube **52** is configured for evacuation of fecal matter from the colon; tube **53** is configured for sensing, such as sensing of pressure in the colon, for example by having one or more pressure sensors **500** configured at a distal portion of the tube; and tube **54** is configured for irrigation of the colon, and may comprise jet-forming distal head (not shown in this figure). Optionally, the proximal ends of tubes **52, 53** and **54** are connected to a work station (not shown in this figure).

In some embodiments, colonoscope **50** is received within lumen **14** of inflated inner sleeve **11.** Optionally, in an inflated state, a diameter **502** of lumen **14** along at least some segments of inner sleeve **11** is at least 5%, 10%, 20%, 40% or intermediate, larger or smaller percentages larger than a diameter **504** of colonoscope **50.** For example, in the inflated state, diameter **502** ranges between 13-25 mm, such as 14 mm, 22 mm, 24 mm or intermediate, larger or smaller diameters.

In some embodiments, for example prior to inflation, a cap **51** is placed over a distal opening of inner sleeve **11.** Optionally, cap **51** seals lumen **14** during inflation, so that the inserted air causes a radially outward expansion of the walls **506** of sleeve **11.** In some embodiments, once colonoscope **50** is positioned within inner sleeve **11,** cap **51** is removed, allowing air to exit the sleeve and/or enabling the advancement of a distal head of colonoscope **50** until it is aligned with the distal opening of the sleeve or protrudes externally from the distal opening.

In some embodiments, cap **51** is coupled to the sleeve by a threaded connection, and is unscrewed from the sleeve. Additionally or alternatively, cap **51** is attached by a breakable connection, and is broken off to allow air to exit the sleeve.

In some embodiments, the radial expansion, for example obtained by inflation, is non symmetrical. For example, a circumferential portion of the inner sleeve **11** that is not attached to outer sleeve **10** may expand more than the attached portion.

In some embodiments, a housing **30** is configured at a distal end of the sleeve assembly. Optionally, housing **30** encases the distal ends of tubes **52, 53** and **54,** and/or the distal end of colonoscope **50.** Optionally, housing **30** encases the distal openings of inner sleeve **11** and outer sleeve **10.** In some embodiments, housing **30** aligns the distal ends of tubes **52, 53** and **54** with respect to the distal head of colonoscope **50.** Optionally, housing **30** comprises one or more extensions **31,** for example extending beyond a distal opening of lumen **14** of the sleeve, for example to a distance ranging between 0.1 mm-10 cm beyond a distal opening of lumen **14.**

Optionally, extension **31** reduces or prevents damage to tissue such as colon wall tissue when the colonoscope is in the colon.

In some embodiments, housing **30** is made of a flexible material, for example polyurethane. In some embodiments, housing is formed of a material having shore hardness value ranging between 40-90, such as 50, 70, 85 or intermediate, larger or smaller values. A potential advantage of a flexible housing **30** may include reducing damage to tissue such as the walls of the colon during insertion of the colonoscope and attached add-on tubes. Another potential advantage of a flexible housing may include inflating the inner sleeve without having to remove the housing, as the elasticity of housing **30** provides for expansion. Alternatively, in some embodiments, distal housing **30** is rigid.

In some embodiments, an external sleeve **508** is positioned over the sleeve assembly, for example during inflation. Optionally, external sleeve **508** is formed of a rigid material, such as hardened polyurethane. Potentially, this prevents over-expansion of the sleeve assembly. For example, external sleeve **508** may prevent over-expansion of outer sleeve **10** which is, in some embodiments, caused to expand as a result of inflation of inner sleeve **11.** Optionally, external sleeve **508** maintains a total diameter **510** of the sleeve assembly under, for example, 25 mm, 20 mm, 30 mm, or intermediate, larger or smaller diameters. Optionally, external sleeve **508** is removed before insertion of the sleeve assembly to the colon, for example after collapsing.

In some embodiments, one or more tubes **52, 53** and/or **54** are held to colonoscope **50,** for example, at one or more longitudinal segments of the colonoscope, by an arrangement other than the sleeve. For example, the tubes are attached to the colonoscope using tape, for example tape wrapped around both the colonoscope and the tubes to hold them together. In some embodiments, the sleeve assembly holds the tubes to the colonoscope along a first longitudinal segment of the sleeve, and other fastening means such as tape couple between the colonoscope and the tubes along a second longitudinal segment. In an example, a distal portion of colonoscope **50** extending from a distal end in a proximal direction to, for example, 5%, 10%, 15% of the colonoscope's length is attached to the tubes using one or more tape wraps, and the rest of the colonoscope is coupled to the tubes by the sleeve assembly.

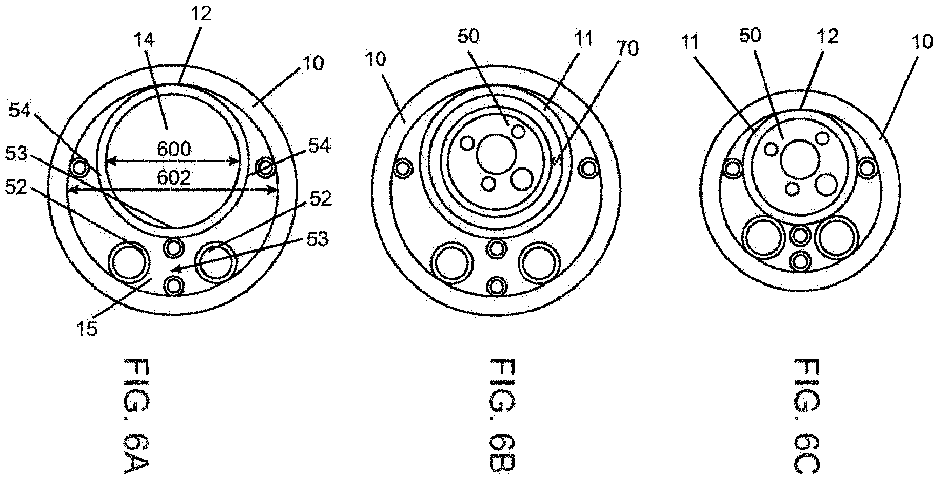

*Figures 6A-C* are drawings of a set up procedure for coupling between a colonoscope and one or more add-on tubes using a sleeve assembly, according to some embodiments of the invention.

*Figure 6A* shows a cross section of the sleeve assembly before insertion of colonoscope **50** through. Add-on tubes **52, 53** and **54** are configured within lumen **15,** between inner sleeve **11** and outer sleeve **10.** In this example, a set of each type of tube is shown (i.e. two evacuation tubes **52,** two sensing tubes **53,** and two irrigation tubes **54).** Alternatively, different amounts and/or different types of tubes may be used. The configuration for example as shown in *figure 6A* shows lumen **14** of inner sleeve **11** before inflation.

*Figure 6B* shows an inflated lumen **14** of inner sleeve **11.** A radial gap **70** is formed between an original location of the wall of inner sleeve **11,** for example with respect to a wall of outer sleeve **10,** as shown for example in *figure 6A* and indicated by the dashed circle in *figure 6B**,* and a location of the wall of inner sleeve **11** after inflation. Optionally, radial gap **70** ranges between, for example, 0.2-8 mm. In some embodiments, colonoscope **50** is advanced through lumen **14** of inflated inner sleeve **11.** Optionally, the distanced walls of inflated inner sleeve **11** provide for smooth delivery of the colonoscope through the lumen.

In some cases, radially outward force is exerted on the add-on tubes and/or on the wall of outer sleeve **10** by the walls of inflated inner sleeve **11.** Optionally, outer sleeve **10** is caused to expand radially as a result of the applied force.

*Figure 6C* is a cross section of the sleeve assembly following collapsing. The wall of inner sleeve **11** is closely fitted on colonoscope **50.** Gap **70** is reduced, for example reduced to fit a diameter of colonoscope **50,** such as a 12 mm diameter, 9 mm diameter, 15 mm diameter, or intermediate, larger or smaller diameters. Optionally, gap **70** is fully reduced, and a diameter **600** of lumen **14** of inner sleeve **11** returns to its initial size, for example as shown in *figure 6A**.* Optionally, gap **70** is fully reduced, and a diameter **600** of lumen **14** of collapsed inner sleeve **11** is further reduced to a size smaller than its initial size, for example 2-5 mm smaller than its initial size.

In some embodiments, add-on tubes **52, 53** and **54** are held by outer sleeve **10** radially closer to colonoscope **50.** Optionally, collapsing increases a contact area between the wall of inner sleeve **11** and the wall of outer sleeve **10,** for example a circumferential segment of coupling **12** is effectively lengthened.

Optionally, for example due to the elasticity of outer sleeve **10,** collapsing of inner sleeve **11** causes the wall of outer sleeve **10** to contract. Optionally, a diameter **602** of lumen **15** of outer sleeve **10** is reduced with respect to its initial position, for example as shown in *figure 6A**,* for example reduced by 2-5 mm, 0.2-4 mm, 2-5 mm, or intermediate, larger or smaller ranges.

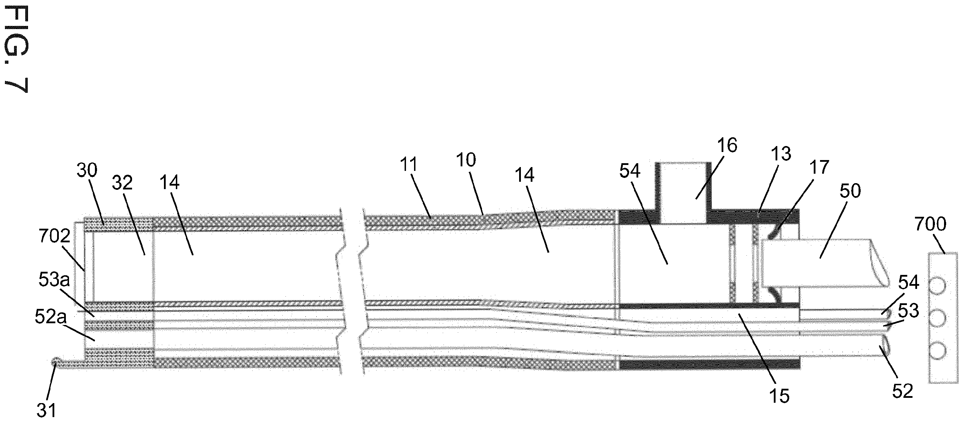

*Figure* 7 shows a colonoscope coupled to one or more add-on tubes by a sleeve, according to some embodiments of the invention.

In some embodiments, a proximal end of tubes **52, 53** and **54** is connected to a workstation **700.** Optionally, workstation **700** comprises, for example, one or more of a liquid tank for supplying liquid such as water to irrigation tube **54,** a vacuum source (or a connection to an external vacuum source) for supplying vacuum to evacuation tube **52,** circuitry for example for acquiring data from one or more sensors of sensing tube **53.** Optionally, the add-on tubes extend in the proximal direction along the colonoscope's umbilical cord (not shown in this figure), until reaching workstation **700.** In some embodiments, the add-on tubes attached at one or more longitudinally distributed locations to the umbilical cord, for example by a tape wrap, a clasp, or other attachment means, to prevent them from interfering with handle movement or the like.

In some embodiments, the distal ends **52a, 53a** of the add-on tubes are aligned with a plurality of distal openings in distal housing **30.** Optionally, distal ends **52a, 53a** are aligned with a distal head **702** of the colonoscope. Additionally or alternatively, housing **30** is constructed to hold one or more add-on tubes beyond head **702** of the colonoscope in a distal direction, and/or to hold one or more add-on tubes short of head **702** in a proximal direction.

Optionally, distal extension **31** protrudes to a distance beyond the distal openings of housing **30.**

In some embodiments, colonoscope **50** is advanced towards a distal end of sleeve **11.** Optionally, the colonoscope is advanced until a head **702** configured at a distal end of the colonoscope is aligned with a distal opening of distal housing **30,** for example as shown herein. Additionally or alternatively, the colonoscope is advanced until reaching a cap positioned at a distal end of the sleeve. Additionally or alternatively, the colonoscope is advanced until colonoscope head **702** protrudes externally from a distal opening of the sleeve.

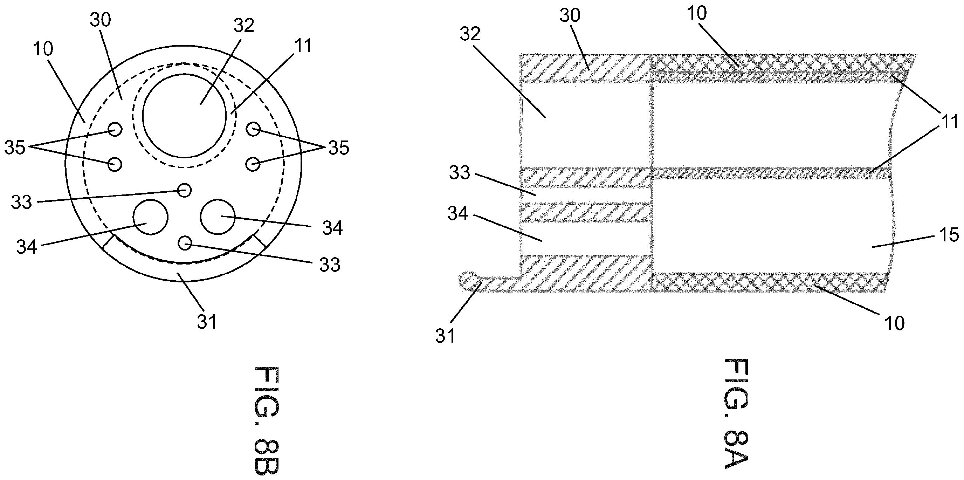

*Figures 8A-8B* show a side view *(**Figure* 8*A*) and front view (*Figure 8B*) of a distal housing of a sleeve assembly, according to some embodiments of the invention. In some embodiments, housing **30** is formed with a plurality of distal openings, for example opening **32** for delivering a colonoscope, and/or openings **33, 34** and/or **35** (shown for example in *figure 8B*) for delivering add-on tubes. Optionally, a distal opening is formed with a circular profile, an elliptic profile, a square profile, a rectangular profile, a triangular profile or any other shape suitable compatible with a distal end of an add-on tube or a distal end of the colonoscope.

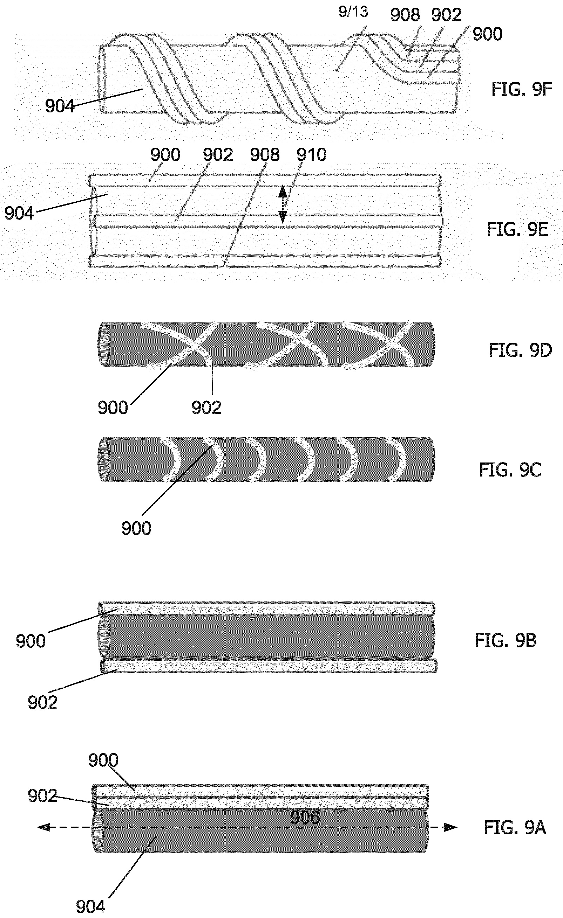

*Figures 9A-9F* show various schematic configurations for positioning one or more add-on tubes with respect to a colonoscope, according to some embodiments of the invention.

In some embodiments, one or more add tubes **900, 902** are oriented with respect to a colonoscope **904.** Optionally, the tubes are positioned with respect to a longitudinal axis **906** of the colonoscope. Additionally or alternatively, the tubes are oriented with respect to a handle of the colonoscope.

The exemplary schematic configurations shown herein may correspond with a collapsed configuration of the sleeve, in which the tubes are held in proximity the colonoscope. Various embodiments may include a different number of add-on tubes, and/or different orientation configurations with respect to the colonoscope.

*Figure 9A* shows tubes **900, 902** aligned in parallel to longitudinal axis **906** of the colonoscope, the tubes being positioned on a first side of axis **906,** according to some embodiments.

*Figure 9B* shows tubes **900, 902** aligned in parallel to longitudinal axis **906** of the colonoscope, the tubes positioned on opposite sides of axis **906,** according to some embodiments.

*Figure 9C* shows tube **900** helically wound around colonoscope **904,** according to some embodiments.

*Figure 9D* shows tubes **900** and **902** interlaced with each other and wrapped around the colonoscope, according to some embodiments.

*Figure 9E* shows a plurality of tubes such as tubes **900, 902, 908** arranged circumferentially around colonoscope **904.** Optionally, the tubes are equally spaced from each other, with a distance **910** between them. Alternatively, the tubes are arranged with varying distances between them.

*Figure 9F* shows a plurality of tubes such as **900, 902** and **908** spirally wounded around colonoscope **904.** A potential advantage of spirally winded tubes may include facilitating insertion into the colon, as often the physician rotates the colonoscope around its axis, and spirally winded tubes would reduce the resistance to rotation, for example as compared to parallel extending tubes.

In some embodiments, the one or more tubes are arranged to least affect the steering of the colonoscope. Optionally, there is a pre-defined orientation of the handle with respect to the insertion tube of the colonoscope, and the tubes are positioned to minimize their effect on, for example, steering of the colonoscope, for example by being wound around the colonoscope.

In some embodiments, for example when a sleeve assembly comprising an inner sleeve and an outer sleeve is used, an inner surface of the outer sleeve defines one or more elongated grooves or paths in which the add-on tubes are received. Optionally, the grooves are parallel to the longitudinal axis of the inner sleeve, so that the add-on tubes are aligned in parallel to the colonoscope that is received within the inner sleeve. Additionally or alternatively, the grooves extend at an angle (e.g. twisted around) and/or are otherwise arranged with respect to the inner sleeve.

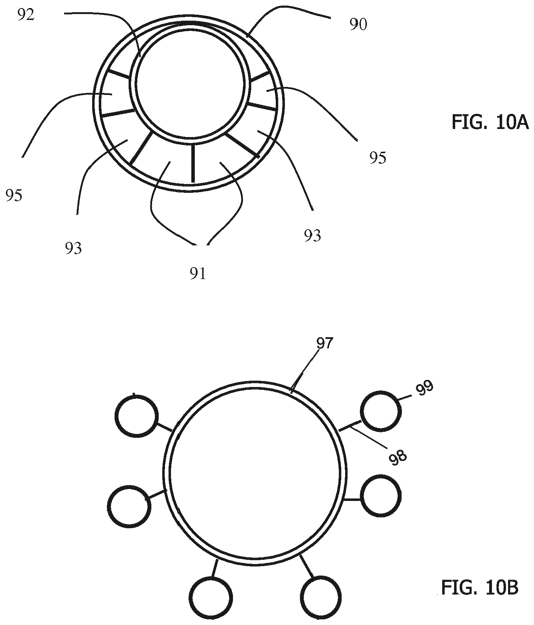

*Figures 10A-10B* are various alternative configurations of a sleeve, according to some embodiments of the invention.

*Figure 10A* shows a cross section of an outer sleeve **90** encompassing an inner sleeve **92.** In some embodiments, a lumen between outer sleeve **90** and inner sleeve **92 is** divided into a plurality of compartments such as **93, 94,** and **95.** Optionally, one or more walls and/or other types of barriers are positioned between the inner and outer sleeves to divide the lumen into compartments. In some embodiments, compartments **93, 94** and/or **95** extend axially along the sleeve, thereby potentially being coaxial with a colonoscope positioned within inner sleeve **92.**

In some embodiments, the compartments are functionally equivalent to a set of add-on tubes. In an example, compartment **91** is used for evacuation of matter from the colon; compartments **93** are used for sensing; compartments **95** are used for irrigation.

*Figure 10B* shows a cross section of a sleeve **97** comprising a plurality of extensions **98,** each extension leading to one or more lumens **99,** for example configured as tubes. In some embodiments, extensions **98** are arranged circumferentially, extending from sleeve **97** in a radially outwards direction.

Optionally, a lumen **99** that is coupled to sleeve **97** by an extension **98** extends longitudinally along inner sleeve **97.** In some embodiments, lumen **99** serves for one or more of, for example, irrigation, sensing, and/or evacuation from the colon, alongside a colonoscope that is positioned within sleeve **97.**

Optionally, such as in the configuration described herein, a need for an outer sleeve is reduced, as the lumens **99** are held to inner sleeve **97** by extensions **98.**

In some embodiments, an extension **98** is rigid. Alternatively, extension **98** is elastic. In some embodiments, extensions **98** can be folded towards the center of sleeve **97,** for example to a position in which tubes **99** are moved to fit closely around the sleeve. Folding and/or twisting the extensions relative to the sleeve to bring the add-on tubes radially closer to the sleeve may be advantageous when inserting the sleeve assembly, comprising the colonoscope and add-on tubes, into the colon. In some embodiments, extensions **98** bounce back to an extended position when inside the colon.

In some embodiments, a length of an extension **98** is selectable and/or controllable. Optionally, extension **98** is structured as a telescopic pole, providing for distancing or approximating lumen **99** to or from sleeve **97.**Optionally, a length of extension **98** ranges between, for example, 0.5 mm to 20 mm, such as 2 mm, 10 mm, 15 mm, or intermediate, longer or shorter lengths. In some embodiments, extensions **98** vary in length.

In some embodiments, for example if extension **98** is in the form of a cable, a diameter of the cable may range between, for example, 2-5 mm.

In some embodiments, configurations for example as described herein are manufactured using an extrusion technology.

In some embodiments, extension **98** is formed of a similar material as sleeve **97** is formed of, for example comprising polyurethane.

In some embodiments, a tube defining lumen **99** is formed of a soft material, to reduce damage to the walls of the colon. Optionally, the tubes are coated by a lubricating material, such as Vaseline, to smoothly glide into the colon.

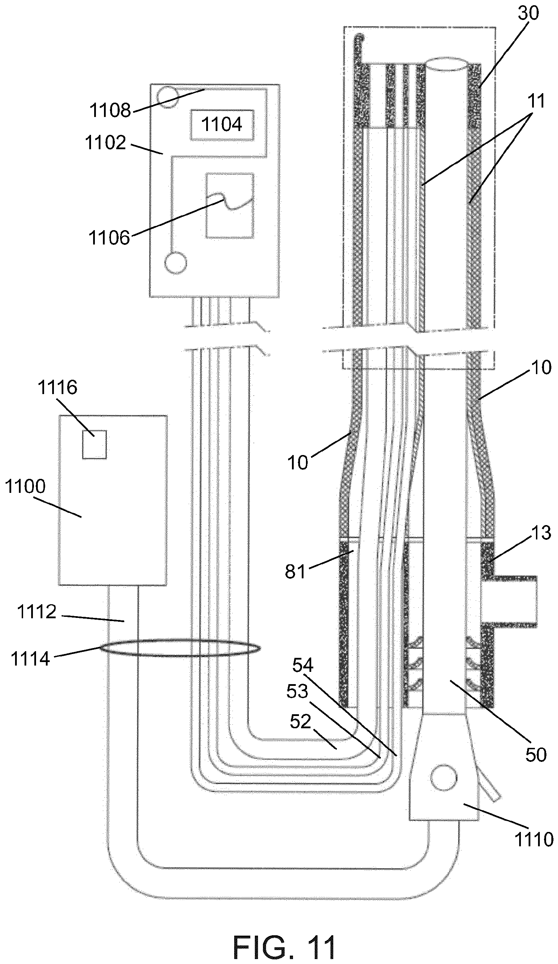

*Figure 11* shows a colonoscopy system in which the colonoscope's insertion tube is coupled to a plurality of add-on tubes by a sleeve, according to some embodiments of the invention.

In some embodiments, a colonoscopy system comprises a colonoscope **50** coupled, on a proximal end, to a colonoscope workstation **1100.** In some embodiments, workstation **1100** comprises a controller **1116,** adapted for performing one or more function associated with operation of colonoscope **50.** In some embodiments, controller **1116** is configured to activate and/or perform functions such as one or more of detecting pressure changes, detecting a clog in a colonoscope, assessing changes in flow in the colonoscope, activating evacuation of fecal matter, activating irrigation of the colon with fluids and/or gas, and/or other functions involved with operation of the colonoscope.

As further shown in this figure, a set of add-on tubes such as tubes **52, 53** and/or **54** are coupled to colonoscope **50** by a sleeve, including, for example, an inner sleeve **11** in which the colonoscope is received, and an outer sleeve **10** holding the add-on tubes to the colonoscope. In some embodiments, the add-on tubes are attached, on their proximal ends, to a workstation **1102.** Optionally, the add-on tubes are operated through workstation **1102.**

In some embodiments, workstation **1102** comprises a vacuum source **1104** and/or one or more liquid tanks **1106,** which are connectable to the add-on tubes. In some embodiments, workstation **1102** comprises circuitry **1108,** such as circuitry configured for operating the add-on tubes, for example configured for activating and/or ceasing vacuum through the one or more tubes. In some embodiments, the circuitry is configured for acquiring data from one or more sensors, such as sensors configured within and/or mounted externally to the add-on tubes and/or the colonoscope.

In some embodiments, colonoscope workstation **1100** and add-on tube workstation **1102** are physically and/or operatively combined together to a single workstation.

In some embodiments, a handle **1110,** adapted for operation by a user such as a physician, is configured between the insertion tube of colonoscope **50** and the umbilical cord **1112.** Alternatively, the handle is configured along the length of colonoscope **50** at a different location.

In some embodiments, the add-on tubes are connected, for example in proximity to their proximal ends, to the colonoscope's umbilical cord **1112.**

Optionally, the add-on tubes are tied to the umbilical cord by a connecting structure such as a cable **1114.** In some embodiments, a clasp, tape, or any other element suitable for attaching the add-on tubes to the colonoscopy is used. A potential advantage of coupling between the add-on tubes and the umbilical cord of the colonoscope may include reducing interfering of the add-on tubes during operation, facilitating maneuvering of the colonoscope by a physician.

In some **embodiments,** one or more additional coupling structures such as proximal housing **13** and/or distal housing **30** provide a connection between the add-on tubes and the colonoscope at various locations along the length of the add-on tubes and/or colonoscope, optionally in addition to the coupling provided by the sleeve.

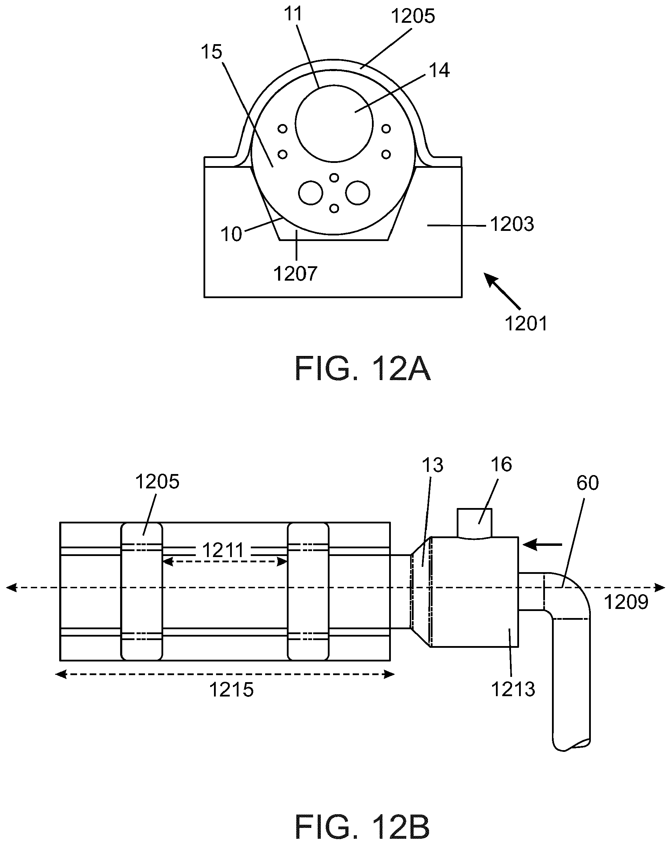

*Figures 12A-B* illustrate an exemplary track jig **1201** for setting up the colonoscope and add-on tubes assembly, according to some embodiments of the invention. In some embodiments, a jig **1201** comprises a base portion **1203,** and one or more fasteners **1205** for holding the sleeve assembly to the base portion during set up.

In some embodiments, base portion **1203** is formed as an elongated, linear track, defining a recess **1207** in which the sleeve is received. Optionally, for example as shown in the transverse cross section of the jig in *figure 12A**,* recess **1207** comprises a substantially trapezoidal cross section profile, but may be otherwise shaped, for example having a semi-circle cross section profile, a rectangular cross section profile, or other profile suitable for receiving the sleeve assembly.

In some embodiments, the sleeve assembly, for example comprising an inner sleeve **11,** in which a colonoscope is received, and an outer sleeve **10** which encompasses the one or more add-on tubes, is introduced into recess **1207** of the jig, and is positioned to extend lengthwise within recess **1207.**

In some embodiments, outer sleeve **10** and/or inner sleeve **11** are inflated, for example by connecting an air pump to deliver air through opening **16,** configured, for example, at a proximal housing **13** of the sleeve assembly. In some embodiments, outer sleeve **10** is inflated until its radial and/or axial expansion is restricted by the one or more fasteners **1205.**

In some embodiments, a colonoscope **50** is introduced into the sleeve assembly, for example through a proximal opening **1213** of housing **13.** In some embodiments, colonoscope **50** is advanced within lumen **14** of inner sleeve **11,** in a way that it lays coaxially to jig **1201.** A potential advantage of threading the colonoscope through a sleeve assembly which is held firmly by the jig may include facilitating advancing the colonoscope through, as the sleeve is restrained from moving during insertion.

Providing a temporary fixation of the sleeve combined with inflating the sleeve to reduce friction during insertion may accelerate threading of the colonoscope into the sleeve, potentially reducing preparation time before the colonoscopy procedure.

In some embodiments, fasteners **1205** extend transversely over recess **1207.** In some embodiments, fasteners **1205** are suitable to keep the sleeve assembly from moving out of recess **1207.** Additionally or alternatively, fasteners **1205** restrict an extent of inflation of the sleeve assembly. Additionally or alternatively, fasteners **1207** limit axial movement of the sleeve assembly within recess **1207,** for example by a textured inner surface of a fastener **1207,** facing the colonoscope, which is effective to increase friction between the sleeve and the fastener. Optionally, the textured inner surface of fastener **1207** comprises, for example, bumps, protrusions, and/or other roughening texture. Alternatively, some movement of the sleeve assembly, such as axial back and forth movement, may be allowed by the fasteners.

In some embodiments, fasteners **1205** include one or more of, for example, clasps, bars, bands, and/or other types of fasteners suitable to hold the sleeve assembly in the jig.

In some embodiments, fasteners **1205** are elastic, and can be stretched for example when the sleeve assembly (inner sleeve **11** and/or outer sleeve **10)** is inflated. Alternatively, fasteners **1205** are rigid.

In some embodiments, fasteners **1205** are distributed along a longitudinal axis **1209** of jig **1201.** Optionally, the fasteners are equally spaced, for example two fasteners are positioned at a distance **1211** ranging between, for example, 10-30 mm, 5-15 mm, 20-50 mm, or intermediate, larger or smaller ranges from each other.

Alternatively, the fasteners are not equally spaced. In some embodiments, a number of fasteners **1205** and/or a spacing between the fasteners is selected such as to reduce or prevent movement of the sleeve held by the jig.

In some embodiments, jig **1201** is continuous. Alternatively, jig **1201** is non-continuous, for example supporting the sleeve assembly at a plurality of points and/or segments along a longitudinal axis of the sleeve.

In some embodiments, a length **1215** of jig **1201** matches a length of colonoscope **50,** for example ranging between 1-3 m, such as 1.2 m, 1.7 m, 2.5 m or intermediate, longer or shorter lengths. A potential advantage of an elongated jig may include facilitating insertion of the colonoscope into the sleeve, for example because the jig supports the sleeve at a plurality of points along the length of the sleeve, enabling fast alignment of the colonoscope.

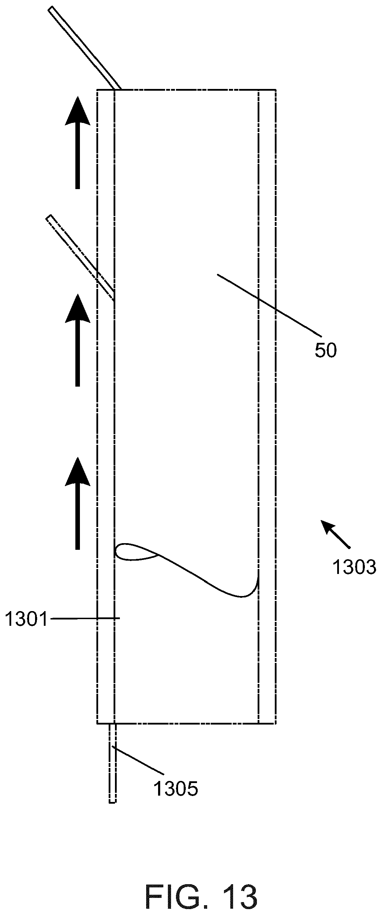

*Figure 13* illustrates an exemplary mechanism for detaching a colonoscope from a sleeve assembly, for example after completion of the colonoscopy procedure, according to some embodiments of the invention.

In some embodiments, a colonoscope **50** is separated from the add-on tubes, for example after the colonoscope and tubes have been removed from the body. In some embodiments, the add-on tubes are disposed of, and the colonoscope is cleaned, disinfected, and/or otherwise prepared for additional use.

In some embodiments, the separation process of the colonoscope from the add-on tubes is performed in a manner that does substantially damage the colonoscope, for example does not scratch or tear the outer layer of the colonoscope.

In the exemplary mechanism shown in *figure 13**,* a string or wire **1301** is embedded in the sleeve assembly **1303.** In some embodiments, wire **1301** extends axially along the sleeve. In some embodiments, by pulling on a free end of the wire **1305,** for example from a distal point in the sleeve in a proximal direction, the wire tears open the sleeve and the colonoscope can be easily removed.

In some embodiments, for example if sleeve assembly **1303** comprises an outer sleeve and an inner sleeve, wire **1301** may be positioned along the inner and/or the outer sleeve. In an example, a first wire embedded in the outer sleeve is pulled to tear open the outer sleeve, thereby separating the add-on tubes from the colonoscope, and a second wire embedded in the inner sleeve in which the colonoscope is positioned is then pulled to tear open the inner sleeve and release the colonoscope. Additionally or alternatively, the inner and/or outer sleeve are cut open, for example using scissors or a knife. Additionally or alternatively, the inner and optionally the outer sleeve as well are re-inflated, and the colonoscope is pulled out in a similar manner to its insertion to the sleeve.

The terms "comprises", "comprising", "includes", "including", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular form "a", "an" and "the" include plural references unless the context clearly dictates otherwise. For example, the term "a compound" or "at least one compound" may include a plurality of compounds, including mixtures thereof.

Throughout this application, various embodiments of this invention may be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range.

Whenever a numerical range is indicated herein, it is meant to include any cited numeral (fractional or integral) within the indicated range. The phrases "ranging/ranges between" a first indicate number and a second indicate number and "ranging/ranges from" a first indicate number "to" a second indicate number are used herein interchangeably and are meant to include the first and second indicated numbers and all the fractional and integral numerals therebetween.

As used herein the term "method" refers to manners, means, techniques and procedures for accomplishing a given task including, but not limited to, those manners, means, techniques and procedures either known to, or readily developed from known manners, means, techniques and procedures by practitioners of the chemical, pharmacological, biological, biochemical and medical arts.

As used herein, the term "treating" includes abrogating, substantially inhibiting, slowing or reversing the progression of a condition, substantially ameliorating clinical or aesthetical symptoms of a condition or substantially preventing the appearance of clinical or aesthetical symptoms of a condition.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

## Claims

1. A sleeve assembly for coupling between an endoscope insertion tube and one or more add-on tubes, the sleeve assembly comprising:
an inner sleeve defining an elongated lumen with an open end, and an having an expanded state sized to surroundingly receive at least 70% of a length of an endoscope insertion tube arranged longitudinally within the inner sleeve;
an outer sleeve encircling the inner sleeve; and
one or more add-on tubes extending longitudinally between the inner sleeve and the outer sleeve;
wherein inner sleeve is collapsible from the expanded state to fit tightly over the received length of the endoscope insertion tube; and
**characterized in that** collapsing the inner sleeve from the expanded state brings the outer sleeve radially closer to the endoscope while allowing at least some movement of the add on tubes in respect to said endoscope.

2. The sleeve assembly according to claim 1, wherein said endoscope comprises a colonoscope.

3. The sleeve assembly according to any one of claims 1 and 2, wherein a distal end of said sleeve assembly comprises a housing.

4. The sleeve assembly according to claim 3, wherein said housing encases distal ends of at least one add-on tube and/or the distal end of an endoscope.

5. The sleeve assembly according to any one of claims 3 and 4, wherein said housing encases distal openings of at least one of said inner sleeve and said outer sleeve.

6. The sleeve assembly according to any one of claims 3-5, wherein said housing aligns distal ends of said add-on tubes with respect to a distal head of an endoscope.

7. The sleeve assembly according to any one of claims 3-6, wherein said housing comprises one or more extensions.

8. The sleeve assembly according to claim 7, wherein said extensions extend beyond a distal opening of a lumen of at least one of said sleeves.

9. The sleeve assembly according to any one of claims 7 and 8, wherein said extensions extend a distance ranging between 0.1 mm-10 cm beyond a distal opening of a lumen of at least one of said sleeves.

10. The sleeve assembly according to any one of claims 7-9, wherein said extensions are configured to at least reduce damage to tissue when an endoscope is in the colon.

11. The sleeve assembly according to any one of claims 1-10, wherein the inner sleeve and the outer sleeve have the same elasticity.

12. The sleeve assembly according to any one of claims 1-10, wherein the sleeve assembly comprises a valve configured to inflate said inner sleeve with air.

13. The sleeve assembly according to any one of claims 1-12, wherein a proximal end of said sleeve assembly comprises a housing structured to couple between said sleeve assembly and said endoscope's handle.

14. The sleeve assembly according to claim 13, wherein said housing is rigid and transfers torsion towards a distal end of the sleeve assembly.

15. The sleeve assembly according to any one of claims 13 and 14, wherein said housing comprises one or more openings that provide passage for fluid delivered for inflating said inner sleeve.
